# EUROPEAN PATENT SPECIFICATION

(11) **EP 2 654 417 B1**
(45) Date of publication and mention of the grant of the patent: **11.07.2018**
(21) Application number: 11807839.3
(22) Date of filing: 21.12.2011
(51) Int. Cl.: A01N 1/02

(54) **CRYOPROTECTIVE COMPOSITIONS AND USES THEREOF**
KRYOPROTEKTIVE ZUSAMMENSETZUNGEN UND DEREN VERWENDUNG
COMPOSITIONS CRYOPROTECTRICES ET LEUR UTILISATION

(30) Priority: 23.12.2010 US 201061426645 P; 23.12.2010 EP 10196808
(43) Date of publication of application: 30.10.2013
(73) Proprietor: DuPont Nutrition Biosciences ApS, 1411 Copenhagen K (DK)
(72) Inventor: HOLLARD, Christophe, McFarland, Wisconsin 53558 (US); FETT, David, Monona, Wisconsin 53716 (US); WEXØE PETERSEN, Lars, Muskego, Wisconsin 53150 (US)
(74) Representative: DuPont EMEA
(86) International application number: PCT/US2011/066461
(87) International publication number: WO 2012/088261

(56) References cited:
- EP-A1- 1 186 235
- US-A1- 2007 105 186
- PORTNER ET AL: "Optimising the viability during storage of freeze-dried cell preparations of Campylobacter jejuni", CRYOBIOLOGY, ACADEMIC PRESS INC, US, vol. 54, no. 3, 13 May 2007 (2007-05-13), pages 265-270, XP022077867, ISSN: 0011-2240, DOI: 10.1016/J.CRYOBIOL.2007.03.002

## Description

### FIELD OF THE INVENTION

The invention relates to Cryoprotective compositions and uses thereof.

### BACKGROUND OF THE INVENTION

The activity, the viability and long term preservation of biological material, in particular microorganisms and eukaryote cells, and of active molecules, e.g. enzymes, may be affected by a number of environmental factors, for example temperature, pH, the presence of water and oxygen or oxidizing or reducing agents. Generally, biological material and active molecules, and especially microorganisms must be subjected to a preservation process for their long-term conservation, e.g. must be dried, frozen or freeze-dried, before or during mixing with other foodstuff ingredients or for direct consumption as dietary supplements. These preservation processes can often result in a significant loss in activity and viability from mechanical, chemical, and osmotic stresses induced by the preservation process. In addition, loss of activity and viability can occur at many other distinct stages, e.g. drying during a food product manufacturing, feed preparation (high temperature and high pressure), transportation and long term storage (temperature and humid exposure), etc. Manufacturing food or feedstuffs with living material is particularly challenging, because the living organisms are very sensitive to preservation processes and to temperature and moisture conditions of the food or feedstuff.

As a result, most of the biological materials or active molecules lose viability or activity during the preservation process, the manufacture process, the transport or the storage. To compensate for such loss, an excessive quantity of biological material or active molecules is included in the product in anticipation that only a portion will survive or remain active. In addition to questionable shelf-life viability for these products, such practices are certainly not cost-effective. Various protective agents have thus been used in the art, with varying degrees of success. These include proteins, certain polymers, skim milk, glycerol, polysaccharides, and oligosaccharides. Disaccharides, such as sucrose and trehalose (De Antoni, G.L. et al., Cryobology 26, 149-153 (1989); and Leslie, S. B. et al., Applied and Environmental Microbiology, Oct 1995, p. 3592-3597), have also been tested as cryoprotectants.

However, none of the cryoprotectants available to date are satisfactory in terms of preservation of the viability or of the activity of the biological material or active molecules. There is thus a need for new cryoprotectants, conferring an increased viability or an increased preservation of activity to biological material or active molecules subjected to a preservation process, and especially microorganisms US 2007/105186 discloses the preservation of microbial cells using i.a. threhalose and inositol.

### SUMMARY OF THE INVENTION

The invention concerns a synergic composition. The inventors have surprisingly found that a particular combination of sugar(s) and polyol(s), in a specific quantity, increases the viability and/or preserve the activity of biological material or active molecules subjected to a preservation process, and especially microorganisms.

The invention thus relates to compositions comprising:
(a) a non-reducing sugar, and
(b) inositol or a derivative thereof, wherein said derivative is an inositol in which at least one hydroxyl functions has been modified or substituted,
wherein the w/w ratio (a)/(b) of said compositions is from 1.1 to 1.7, and
(c) microorganisms.

The invention also relates to uses of the compositions according to the invention for preserving microorganisms.

The invention still relates to methods for preserving microorganisms, comprising the following steps:
- preparing a composition according to the invention,
- adding said composition to microorganisms to obtain a composition comprising biological material and/or active molecules,
- submitting said composition comprising microorganisms to at least one preservation step, particularly a freezing, drying or freeze-drying step.

The invention also relates to preserved microorganisms obtainable by a method according to the invention.

The invention further relates to food products, feed products, consumer healthcare products or agri-products comprising a preserved biological material and/or active molecule according to the invention.

### DEFINITIONS

According to the invention, a "non reducing sugar" is any sugar that, in a solution, does not contain a free carbonyl or anomeric carbon, the carbonyl carbon from the aldehyde or ketone group being involved in a glycosidic bond. The non reducing sugar is typically a "non reducing disaccharide" in which the anomeric carbons of the two units are linked together, such as for example sucrose, trehalose, or derivatives thereof.

By "derivative of trehalose" it is meant a compound derived from trehalose by a chemical or physical process, wherein said compound does not contain a free carbonyl or anomeric carbon, the carbonyl carbon from the aldehyde or ketone group being involved in a glycosidic bond.

By "derivative of sucrose" it is meant a compound derived from sucrose by a chemical or physical process, wherein said compound does not contain a free carbonyl or anomeric carbon, the carbonyl carbon from the aldehyde or ketone group being involved in a glycosidic bond.

According to the invention, "inositol" refers to cyclohexane-1,2,3,4,5,6-hexol (C₆H₁₂O₆), which is a sixfold alcohol (polyol) of cyclohexane. "Inositol" according to the invention refers to any one of its possible stereoisomers, such as for example myo-, epi-, scyllo-, chiro-, muco-, allo-, or meso-inositol.

According to the invention, a "derivative of inositol" refers to inositol wherein at least one of the hydroxyl functions has been modified or substituted. Examples of inositol derivatives are phosphated, sulphated or methylated inositol.

According to the invention, a "cryoprotective composition" is a composition which provides to compounds or elements some protection against the harmful effects of low or freezing temperatures, such as the ones submitted for example in freeze-drying or freezing processes. In addition, in the case of freeze-drying or drying, it confers to the dried elements some stability through the drying process. The action of the cryoprotective composition will reduce loss of activity or viability during the manufacturing process and subsequently, its action improves the activity/viability of the biological material or active molecules during storage.

According to the invention, "biological material" refers to material that is capable of self-replication either directly or indirectly. Representative examples include microorganisms (e.g. bacteria, fungi, molds, yeasts, archaea, protists), algae, protozoa, eukaryotic cells, cell lines, hybridomas, plasmids, viruses, plant tissue cells, lichens and seeds.

According to the invention, an "active molecule" refers to any molecule that has an effect on a living tissue. Examples of active molecules are proteins having a biological activity, such as for example enzymes, amino acids, proteins, antibodies.

### DETAILED DESCRIPTION OF THE INVENTION

### Compositions

The inventors have found that the presence of a combination of at least one non-reducing sugar with inositol or a derivative of inositol in a composition comprising biological material and/or active molecules decrease the loss of viability and/or activity of said biological material and/or active molecules generally observed during or after a preservation process. The invention thus relates to a composition comprising:
(a) a non-reducing sugar, and
(b) inositol or a derivative thereof, in which derivative at least one hydroxyl function has been modified or substituted,
wherein the w/w ratio (a)/(b) of said compositions is from 1.1 to 1.7, and
(c) microorganisms.

By using this combination of sugar(s) and polyol(s) in the particular ratio of the invention, an excellent recovery of cells subjected to a preservation process is for instance obtained. Under this specific ratio this combination of sugar(s) and polyol(s) acts as a synergy.

In one embodiment, said composition according to the invention consists of:
(a) a non-reducing sugar, and
(b) inositol or a derivative thereof, in which derivative at least one hydroxyl function has been modified or substituted,
wherein the w/w ratio (a)/(b) of said compositions is from 1.1 to 1.7, and
(c) microorganisms.

The compositions according to the invention preferably have a w/w ratio (a)/(b) from 1.1 to 1.7. According to one embodiment, the compositions according to the invention have a w/w ratio (a)/(b) from 1.2 to 1.7, particularly from 1.3 to 1.7, from 1.4 to 1.6, from 1.45 to 1.55, or more particularly of 1.5.

In another embodiment, in the compositions according to the invention, said (a) is a mixture of non-reducing sugars.

In a particular embodiment, said non-reducing sugar(s) is(are) selected from the group comprising trehalose, derivatives of trehalose, sucrose, and derivatives of sucrose.

In one embodiment, in the compositions according to the invention, (a) is trehalose and (b) is inositol.

### Compositions comprising biological material and/or active molecules

The compositions according to the invention comprise microorganisms, typically selected from bacteria, fungi, molds, yeasts, archaea, protists or any mixture thereof.

In one embodiment, said fungi/molds are selected from *Penicillium spp, Geotrichum spp, Lecanicillium spp,* and *Trichothecium spp.*

In one embodiment, said yeasts are selected from *Kluyveromyces spp, Debaryomyces spp, Yarrowia spp, Pichia spp, Williopsis spp,* and *Saccharomyces spp.*

In a preferred embodiment, said microorganisms are selected from bacteria. Bacteria can be selected from any genus or species. Preferred bacteria according to the invention are lactic acid bacteria, *Bacillus* and coryneform bacteria such as for example *Arthrobacter spp, Corynebacterium spp, Brevibacterium spp.* According to the invention, the term "lactic acid bacteria" includes any bacteria capable of producing, as the major metabolic end product of carbohydrate fermentation, lactic acid or at least one of its derivatives (including, but not limited to, propionic acid). The term is therefore intended to include propionic acid bacteria (PAB), which produce propionic acid as a carbohydrate fermentation product. Preferably the bacteria in the present invention are lactic acid bacteria which are generally recognised as safe for animal or human consumption (i.e. GRAS approved).

Suitable lactic acid bacteria may be selected from the genus *Lactococcus, Lactobacillus, Leuconostoc, Bifidobacterium, Carnobacterium, Enterococcus, Propionibacterium, Pediococcus, Streptococcus* and mixtures thereof. Typically, the microorganisms are probiotics or DFM (Direct Fed Microbials). According to the invention "probiotics" or "DFMs" means live microorganisms which when administered in adequate amounts confer a health benefit to the host, the host being a human in the case of probiotics and an animal in the case of DFMs.

In a particular embodiment, said lactic acid bacteria are selected from the group comprising the strains of the species and subspecies *Bifidobacterium bifidum, Bifidobacterium lactis, Bifidobacterium longum, Bifidobacterium breve, Lactobacillus reuteri, Lactobacillus acidophilus, Lactobacillus casei, Lactobacillus plantarum, Lactobacillus delbruckii bulgaricus, Lactobacillus rhamnosus, Streptococcus thermophilus, Lactococcus lactis, Lactobacillus pentoceus, Lactobacillus buchneri, Lactobacillus brevis, Pediococcus pentosaceus, Pediococcus acidilactici, Pediococcus pervulus, Propionibacterium freudenreichi, Propionibacterium jenseni* and *Streptococcus salivarius.*

In one embodiment, the compositions comprising microorganisms according to the invention comprise microorganisms in a concentration from 1E8 CFU/g of composition to 5E12 CFU/g of composition, particularly from 1E9 CFU/g of composition to 1E12 CFU/g of composition, more particularly from 1E10 CFU/g of composition to 1E11 CFU/g of composition.

In one embodiment, the compositions comprising biological material and/or active molecules according to the invention comprise:
(a) at least 4% by weight of said non-reducing sugar, and
(b) at least 3% by weight of said inositol or a derivative thereof,
wherein the w/w ratio (a)/(b) of said compositions is as defined previously.

In a particular embodiment, the compositions comprising biological material and/or active molecules according to the invention comprise:
(a) at least 5%, particularly at least 6%, 7%, 8%, 9%, 10%, 11%, 12%, 13%, 14%, 15%, or at least 16% by weight of said non-reducing sugar, and
(b) at least 4%, particularly at least 5%, 6%, 7%, 8%, 9%, 10%, 11%, 12%, or at least 13% by weight of said inositol or a derivative thereof,
wherein the w/w ratio (a)/(b) of said compositions is as defined previously.

In another embodiment, the compositions comprising microorganisms according to the invention comprise:
(a) from 4% to 16%, particularly from 4% to 15%, from 4% to 14%, from 4% to 13%, from 4% to 12%, from 5% to 13%, from 6% to 13%, or from 7% to 13% by weight of said non-reducing sugar, and
(b) from 3% to 13%, particularly from 3% to 12%, from 3% to 11%, from 3% to 10%, from 3% to 9%, from 3% to 8%, from 4% to 10%, from 4% to 9%, from 5% to 10%, or from 5% to 9% by weight of said inositol or a derivative thereof,
wherein the w/w ratio (a)/(b) of said compositions is as defined previously.

### Formulation of the compositions according to the invention

The compositions according to the invention can be solid, liquid or under pasty form.

In a preferred embodiment they are in liquid form.

Liquid compositions according to the invention are typically aqueous compositions (e.g. the components are diluted in water).

In addition to the cryoprotective sugar(s) and polyol(s), and the microorganisms, the compositions according to the invention may also comprise other components such as for example other disaccharides (e.g. melibiose, lactulose), oligosaccharides (e.g. raffinose), oligofructoses, polysaccharides (e.g. maltodextrins dextran, PEG, xanthan gum, alginate, pectin, cellulose), pentoses (e.g. ribose, xylose), hexoses (e.g. fructose, mannose, sorbose), salts (phosphate...).

### Applications and methods

The compositions according to the invention are very efficient for preserving the viability or the activity of microorganisms. The invention thus relates to the use of a composition comprising a non-reducing sugar and inositol or a derivative thereof according to the invention for preserving microorganisms. Indeed, thanks to the compositions according to the invention, the viability of the biological material and/or the activity of active molecules is/are enhanced during and after the preservation process.

In one embodiment of the use according to the invention, the composition comprising a non-reducing sugar and inositol or a derivative thereof according to the invention is added to the microorganisms prior to a preservation step, such as a freezing, drying or freeze-drying step.

The compositions according to the invention are also very efficient to reduce hygroscopicity of dried or freeze-dried microorganisms. By hygroscopicity it is meant the tendency of the dried material to absorb water quickly and to change its physical properties when its moisture content increases.

The invention also relates to methods for preserving biological material or active molecules comprising the following steps:
- preparing a composition comprising a non-reducing sugar and inositol or a derivative thereof according to the invention,
- adding said composition to microorganisms to obtain a composition comprising microorganisms,
- submitting said composition comprising microorganisms to at least one preservation step.

The preservation step is preferably a freezing, drying or freeze-drying step. The step of freezing, drying or freeze-drying can be performed according to classical procedures well known by the skilled person. Examples of preservation processes are for example disclosed in the following document: "*bactéries lactiques, de la genetique aux ferments*", George Corrieu and Francois-Marie Luquet, Lavoissier. Preferably, the conservation step is a freeze-drying step.

In a particular embodiment of the uses and methods according to the invention, said microorganisms are under the form of a microorganism concentrate.

A "microorganism concentrate" according to the invention refers to microorganisms that have been submitted to at least a concentration step after their cultivation or synthesis. Some examples of concentration steps are centrifugation, filtration, evaporation, sedimentation or flocculation.

In a particular embodiment, the composition according to the invention is added to the microorganism concentrate in the concentrations as previously defined.

The invention also relates to preserved frozen, dried or freeze-dried microorganisms.

The invention also relates to food products, feed products, consumer healthcare products or agri-products comprising preserved microorganisms obtainable by the methods according to the invention, especially frozen, dried or freeze-dried microorganisms obtainable by the methods according to the invention.

According to the invention, by "food product" it is meant a product or a preparation that is intended to feed a human.

According to the invention, by "feed product" it is meant a product or preparation that is intended to feed an animal.

According to the present invention, "consumer healthcare products" include dietary supplements, nutraceuticals and over-the-counter products. A consumer can be a human and/or an animal.

A dietary supplement (also referred to as a food supplement or nutritional supplement), means a preparation intended to provide nutrients, such as vitamins, minerals, fiber, fatty acids or amino acids, which are missing or are not consumed in sufficient quantity in a person's diet. A dietary supplement can be for human and/or animal consumption. Examples of dietary supplements according to the invention are powder packaged in sachets or sticks, powder incorporated in tablet, powder filled into capsules.

The term "nutraceutical" means a functional food which is capable of providing not only a nutritional effect and/or a taste satisfaction, but is also capable of delivering therapeutic (or other beneficial) effects to the consumer.

An "over-the-counter product" means non prescription medicines which can prevent certain diseases or reduce symptoms associated with gut health or immune health, thereby promoting gut health or improving the immune function. For exemple, these products allow prevention and treatment of allergies, prevention and treatment of respiratory tract infection and other emerging applications of probiotics and direct fed microbials (DFMs).

According to the present invention the expression "agri-product" encompasses biopesticides, biofertilizers, products for plant care, composts and by-products as well as products of bioenergy (bio ethanol, bio ester).

In a particular embodiment of the invention, said product is a food product. More particularly, the food product is a dairy product. Examples of dairy products according to the invention are fermented milk, a yogurt, a matured cream, a cheese, a fromage frais, a milk drink, a dairy product retentate, a processed cheese, a cream dessert, a cottage cheese or an infant milk. Still typically, the dairy product according to the invention comprises milk of animal and/or plant origin.

All the specific and preferred embodiments previously described for the compositions, for example the nature of the microorganisms, the preferred ratios... also applied for these uses, methods and products.

### BRIEF DESCRIPTION OF THE FIGURE

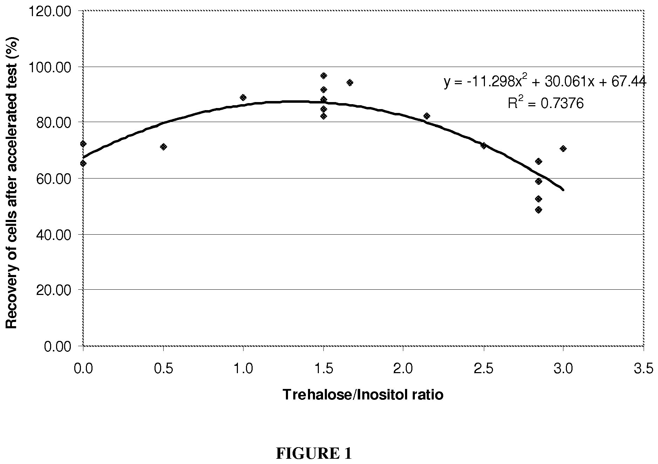

**Figure 1****:** Recovery of cell after accelerated test as a function of Trehalose/Inositol ratio.

### EXAMPLES

### Cells counts measurement

The cell count of the freeze dried material has been evaluated through a typical bacteria enumeration method used for lactic acid bacteria. In this method, the freeze dried bacteria were suspended into a MRS solution with a stomacher and revitalized in that solution for 30 minutes. The suspension was then successively diluted in bottles of peptone buffer and finally cultured on a MRS nutritive media for 48 to 72 hours at 38°C under anaerobic condition. During that period, the bacteria form colonies on the nutritive media. Those colonies were counted and results were expressed as Colony Forming Units (CFU) per gram.

### Accelerated stability test

Long term stability of freeze dried bacteria as a function of time is a critical characteristic for commercial application. The long term stability can be evaluated by accelerated stability test consisting of placing the freeze dried cells into a sealed laminated aluminum foil in a constant temperature chamber maintained at 38°C for fourteen days. The cell count of the freeze dried material is measured before and after the exposure to elevated temperature. A recovery rate of cells is calculated by subtracting the cells measured after the accelerated stability test from the initial cells measurement and dividing the subtraction results by the initial count. The accelerated stability test recovery rate gives a relative estimate of the long term cell stability.

### Example 1

Various cryoprotectants (see table 1) were mixed in a suspension containing, *Lactobacillus acidophilus* at an approximate cell count of 1E11 CFU/gr. The mixture, called stabilized concentrate, was kept at 4-8° C for 1.5 hrs and continuously agitated before being frozen by dispensing droplets of the stabilized concentrate into liquid nitrogen. The resulting frozen droplets are called frozen pellets.

**Table 1: Formulation of Trehalose based cryoprotectant tested on a suspension of Lactobacillus acidophilus.**

| **Cryoprotective solution** | **Trehalose concentration (% W/W of stabilized concentrate)** | **Additional cryoprotective component (% W/W of stabilized concentrate)** |
|---|---|---|
| Trehalose alone experiment 1 | 8 | 0 |
| Trehalose alone experiment 2 | 14 | 0 |
| Trehalose with phosphate | 13 | 1 as KHP04 |
| Trehalose with EDTA | 14 | 0.0021 as EDTA |
| Inositol alone | 0 | 16.7 as Inositol |
| Trehalose with Inositol experiment 1 | 14 | 3.4 as Inositol |
| Trehalose with Inositol experiment 2 | 13 | 6.7 as Inositol |
| Trehalose with Inosine Mono Phosphate (IMP) | 14 | 3.4 as IMP |

The frozen pellets were freeze dried in a Virtis® freeze drier at 100 mT and a cell count measurement was performed just after freeze drying by performing an accelerated stability test.

Table 2 shows the initial cell counts, the cell counts after the accelerated stability test and the recovery of freeze dried cells after the accelerated testing.

**Table 2: Cell counts and accelerated stability results for Trehalose based cryoprotectant tested on a suspension of L. acidophilus.**

| **Cryoprotection solution** | **Viable cell counts after freeze drying (CFU/gr)** | **Viable cell counts after accelerated stability test (CFU/gr)** | **Recovery of viable cell after accelerated stability test (%)** |
|---|---|---|---|
| Trehalose alone experiment 1 | 7.4E+11 | 2.2E+11 | 29.0 |
| Trehalose alone experiment 2 | 6.6E+11 | 2.4E+11 | 38.0 |
| Trehalose with phosphate | 6.4E+11 | 1.3E+11 | 19.9 |
| Trehalose with EDTA | 7.0E+11 | 1.9E+11 | 27.1 |
| Inositol alone | 3.2E+11 | 1.3E+11 | 41.0 |
| Trehalose with Inositol experiment 1 | 5.8E+11 | 3.2E+11 | 54.8 |
| Trehalose with Inositol experiment 2 | 5.0E+11 | 4.4E+11 | 89.0 |
| Trehalose with Inosine Mono Phosphate (IMP) | 5.2E+11 | 1.2E+11 | 23.3 |

Table 2 shows that the best recovery after the accelerated testing is achieved when the trehalose solution is at 13% and the Inositol is at 6.7%, with a ratio Trehalose/Inositol = 1.9.

### Example 2

Various ratio of Inositol over Trehalose have been tested. The cryoprotectants were mixed for 1 to 3 hrs at 10-30°C to a suspension containing *Lactobacillus acidophilus* at an approximate cell count of 1E11 CFU/gr. The mixture was frozen and freeze dried, and a cell count was performed just after freeze drying by performing an accelerated stability test, as disclosed in Example 1. Table 3 gives the results of this testing.

**Table 3: Cell counts and accelerated stability results for Trehalose based cryoprotectant tested on a suspension of L. acidophilus including Trehalose to Inositol ratio and percent recovery of cells after accelerated stability testing.**

| **Trehalose concentration (% W/W of stabilized concentrate)** | **Inositol concentration (% W/W of stabilized concentrate)** | **Ratio Trehalose / Inositol** | **Viable cell counts after freeze drying (CFU/gr)** | **Recovery of viable cell from frozen pellets to freeze dried pellets (%)** | **Viable cell counts after accelerated stability test (CFU/gr)** | **Recovery of viable cells after accelerated stability test (%)** |
|---|---|---|---|---|---|---|
| 6.7 | 4.4 | 1.5 | 4.03E+11 | 104.4 | 3.70E+11 | 91.81 |
| 6.7 | 4.4 | 1.5 | 4.21E+11 | 75.3 | 3.71E+11 | 88.12 |
| 6.7 | 4.4 | 1.5 | 4.14E+11 | 79.3 | 4.01E+11 | 96.86 |
| 6.7 | 4.4 | 1.5 | 4.15E+11 | 90.7 | 3.51E+11 | 84.58 |
| 8.2 | 2.9 | 2.8 | 6.26E+11 | 83.0 | 3.05E+11 | 48.72 |
| 8.2 | 2.9 | 2.8 | 5.95E+11 | 75.2 | 3.50E+11 | 58.82 |
| 8.2 | 2.9 | 2.8 | 6.20E+11 | 87.0 | 3.25E+11 | 52.42 |
| 8.2 | 2.9 | 2.8 | 5.70E+11 | 67.3 | 3.77E+11 | 66.14 |
| 0.0 | 9.1 | 0.0 | 5.14E+11 | 81.2 | 3.72E+11 | 72.37 |
| 12.0 | 8.0 | 1.5 | 4.53E+11 | 82.5 | 3.73E+11 | 82.34 |
| 8.3 | 8.3 | 1.0 | 4.49E+11 | 81.1 | 3.99E+11 | 88.86 |
| 4.3 | 8.7 | 0.5 | 5.33E+11 | 89.0 | 3.80E+11 | 71.29 |
| 12.1 | 7.3 | 1.7 | 4.41E+11 | 87.5 | 4.16E+11 | 94.33 |
| 12.3 | 5.7 | 2.1 | 4.30E+11 | 84.6 | 3.54E+11 | 82.33 |
| 12.4 | 5.0 | 2.5 | 4.86E+11 | 81.9 | 3.48E+11 | 71.60 |
| 12.5 | 4.2 | 3.0 | 4.58E+11 | 81.7 | 3.23E+11 | 70.52 |
| 0.0 | 20.0 | 0.0 | 3.70E+11 | 94.7 | 2.42E+11 | 65.41 |

The recovery of cells after the accelerated stability test were graphed as a function of the Trehalose/Inositol and shown in Figure 1. The curve clearly shows a synergistic effect between Trehalose and Inositol. Initially, the recovery increases when the ratio increases. An optimum is achieved around 1.5, after which the recovery decreases. The best recovery values are obtained when the ratio Trehalose/Inositol is comprised between 1.1 and 1.9.

### Example 3:

Cryoprotectants made of Inositol with non reducing sugar such as Trehalose or Sucrose were mixed with a suspension of *Bifidobacterium lactis* at an approximate cell count of 1E11 CFU/gr. The mixture called stabilized concentrate was kept at 4°C and continuously agitated before being frozen by dispensing droplets of the stabilized concentrate into liquid nitrogen. The resulting frozen droplets are called pellets. The frozen pellets have then been freeze dried in a Virtis® freeze drier under a vacuum at 100 mT. The freeze dried pellets were evaluated by measuring the cells counts just after freeze drying and by performing an accelerated stability test. In addition, the recovery of cells from the frozen pellets to freeze dried pellets was calculated. Tables 4 and 5 give the results of the test.

**Table 4: Comparison of recovery after freeze drying and recovery after an accelerated stability test for a cryoprotectant containing Trehalose and Inositol.**

| **Non-reducing sugar = Trehalose (% W/W)** | **Inositol concentration (% W/W)** | **Ratio Trehalose / Inositol** | **Recovery of viable cell from frozen pellets to freeze dried pellets (%)** | **Recovery of viable cell after accelerated stability test (%)** |
|---|---|---|---|---|
| 8.80 | 6.7 | 1.3 | 91.3 | 84.9 |
| 13.33 | 6.7 | 2.0 | 94.4 | 81.8 |

**Table 5: Comparison of recovery after freeze drying and recovery after an accelerated stability test for a cryoprotectant containing Sucrose and Inositol.**

| **Non-reducing sugar = Sucrose (% W/W)** | **Inositol concentration (% W/W)** | **Ratio Sucrose/ Inositol** | **Recovery of viable cell from frozen pellets to freeze dried pellets (%)** | **Recovery of viable cell after accelerated stability test (%)** |
|---|---|---|---|---|
| 8.80 | 6.7 | 1.3 | 102.0 | 82.5 |
| 13.33 | 6.7 | 2.0 | 91.1 | 84.9 |

The recovery of biomass stabilized with Trehalose or with Sucrose have similar cell recovery after freeze drying or after the accelerated test. Non reducing sugar can be used in combination with Inositol to successfully dry and preserve *Bifidobacterium lactis.*

## Claims

1. A composition comprising:
(a) at least one non-reducing sugar,
(b) inositol or a derivative thereof, wherein said derivative is an inositol in which at least one hydroxyl function has been modified or substituted,
wherein the w/w ratio (a)/(b) of said composition is from 1.1 to 1.7, and
(c) microorganisms.

2. The composition of claim 1, wherein the microorganisms are bacteria.

3. The composition of claim 1, wherein the microorganisms are lactic acid bacteria.

4. The composition according to claim 1, 2 or 3, wherein said w/w ratio (a)/(b) is from 1.3 to 1.7.

5. The composition according to any one of claims 1 to 4, wherein said (a) is a mixture of non-reducing sugars.

6. The composition according to any one of claims 1-5, wherein said non-reducing sugar(s) is(are) selected from the group comprising trehalose, 2,3,2',3'-tetra-O-Benzyl-6,6'-di-O-decanoyl-4,4'-bis-O(diphenylphosphono) alpha, alpha trehalose, 6,6'-di-O-decanoyl-4,4'-di-O-phosphono alpha, alpha trehalose, 2,3,2',3'-tetra-O-benzyl-4,4'-bis-O-(dipheynlphosphono) alpha, alpha trehalose 6,6', fatty acid ester, sucrose, sucrose-6 benzoate, sucrose-6 acetate, sucrose-6 glutarate and sucrose-6 laurate.

7. The composition according to any one of claims 1 to 4, wherein said (a) is trehalose and said (b) is inositol.

8. The composition according to claim 3, wherein said lactic acid bacteria are selected from the group comprising the strains of the genus *Lactococcus, Lactobacillus, Leuconostoc, Bifidobacterium, Carnobacterium, Enterococcus, Propionibacterium, Pediococcus, Streptococcus* and mixtures thereof.

9. The composition according to claim 8, wherein said lactic acid bacteria are selected from the group comprising the strains of the species and subspecies *Bifidobacterium bifidum, Bifidobacterium lactis, Bifidobacterium longum, Bifidobacterium breve, Lactobacillus reuteri, Lactobacillus acidophilus, Lactobacillus casei, Lactobacillus plantarum, Lactobacillus delbruckii bulgaricus, Lactobacillus rhamnosus, Streptococcus thermophilus, Lactococcus lactis, Lactobacillus pentoceus, Lactobacillus buchneri, Lactobacillus brevis, Pediococcus pentosaceus, Pediococcus acidilactici, Pediococcus pervulus, Propionibacterium freudenreichi, Propionibacterium jenseni, Streptococcus salivarius* and mixtures thereof.

10. The composition according to any one of claims 1, 2, 3, 8 and 9, wherein said composition comprises microorganisms in a concentration from 1E8 CFU/g of composition to 5E12 CFU/g of composition.

11. The composition according to any one of claims 1, 2, 3, and 8-10, wherein said composition comprises:
(a) at least 4% by weight of said non-reducing sugar, and
(b) at least 3% by weight of said inositol or a derivative thereof.

12. Use of a composition comprising:
(a) at least one non-reducing sugar,
(b) inositol or a derivative thereof, wherein said derivative is an inositol in which at least one hydroxyl functions has been modified or substituted,
wherein the w/w ratio (a)/(b) of said composition is from 1.1 to 1.7,
for preserving microorganisms.

13. A method for preserving microorganisms, comprising the following steps:
preparing a composition comprising:
(a) at least one non-reducing sugar,
(b) inositol or a derivative thereof, wherein said derivative is an inositol in which at least one hydroxyl function has been modified or substituted,
wherein the w/w ratio (a)/(b) of said composition is from 1.1 to 1.7,
- adding said composition to microorganisms, to obtain a composition comprising microorganisms,
- submitting said composition comprising microorganisms, to a freezing, drying or freeze-drying step.

## Patentansprüche

1. Zusammensetzung umfassend:
(a) mindestens einen nichtreduzierenden Zucker,
(b) Inosit oder ein Derivat davon, wobei es sich bei dem Derivat um ein Inosit, in dem mindestens eine Hydroxyfunktion modifiziert oder substituiert worden ist, handelt,
wobei das w/w-Verhältnis (a)/(b) der Zusammensetzung 1,1 bis 1,7 beträgt, und
(c) Mikroorganismen.

2. Zusammensetzung nach Anspruch 1, wobei es sich bei den Mikroorganismen um Bakterien handelt.

3. Zusammensetzung nach Anspruch 1, wobei es sich bei den Mikroorganismen um Milchsäurebakterien handelt.

4. Zusammensetzung nach Anspruch 1, 2 oder 3, wobei das w/w-Verhältnis (a)/(b) 1,3 bis 1,7 beträgt.

5. Zusammensetzung nach einem der Ansprüche 1 bis 4, wobei es sich bei (a) um eine Mischung von nichtreduzierenden Zuckern handelt.

6. Zusammensetzung nach einem der Ansprüche 1 bis 5, wobei der/die nichtreduzierende(n) Zucker aus der Gruppe umfassend Trehalose, 2,3,2',3'-Tetra-O-benzyl-6,6'di-O-decanoyl-4,4'-bis-O(diphenylphosphono)-alpha,alpha-trehalose, 6,6'-Di-O-decanoyl-4,4'-di-O-phosphono-alpha,alpha-trehalose, 2,3,2',3'-Tetra-O-benzyl-4,4'-bis-O-(diphenylphosphono)-alpha,alpha-trehalose-6,6'-Fettsäureester, Saccharose, Saccharose-6-benzoat, Saccharose-6-acetat, Saccharose-6-glutarat und Saccharose-6-laurat ausgewählt ist/sind.

7. Zusammensetzung nach einem der Ansprüche 1 bis 4, wobei es sich bei (a) um Trehalose und bei (b) um Inosit handelt.

8. Zusammensetzung nach Anspruch 3, wobei die Milchsäurebakterien aus der Gruppen umfassend die Stämme der Gattung *Lactococcus, Lactobacillus, Leuconostoc, Bifidobacterium, Carnobacterium, Enterococcus, Propionibacterium, Pediococcus, Streptococcus* und Mischungen davon ausgewählt sind.

9. Zusammensetzung nach Anspruch 8, wobei die Milchsäurebakterien aus der Gruppe umfassend die Stämme der Spezies und Subspezies *Bifidobacterium bifidum, Bifidobacterium lactis, Bifidobacterium longum, Bifidobacterium breve, Lactobacillus reuteri, Lactobacillus acidophilus, Lactobacillus casei, Lactobacillus plantarum, Lactobacillus delbruckii bulgaricus, Lactobacillus rhamnosus, Streptococcus thermophilus, Lactococcus lactis, Lactobacillus pentoceus, Lactobacillus buchneri, Lactobacillus brevis, Pediococcus pentosaceus, Pediococcus acidilactici, Pediococcus pervulus, Propionibacterium freudenreichi, Propionibacterium jenseni, Streptococcus salivarius* und Mischungen davon ausgewählt sind.

10. Zusammensetzung nach einem der Ansprüche 1, 2, 3, 8 und 9, wobei die Zusammensetzung Mikroorganismen in einer Konzentration von 1E8 KBE/g Zusammensetzung bis 5E12 KBE/g Zusammensetzung umfasst.

11. Zusammensetzung nach einem der Ansprüche 1, 2, 3 und 8-10, wobei die Zusammensetzung Folgendes umfasst:
(a) mindestens 4 Gew.-% des nichtreduzierenden Zuckers und
(b) mindestens 3 Gew.-% des Inosits oder eines Derivats davon.

12. Verwendung einer Zusammensetzung, die Folgendes umfasst:
(a) mindestens einen nichtreduzierenden Zucker,
(b) Inosit oder ein Derivat davon, wobei es sich bei dem Derivat um ein Inosit, in dem mindestens eine Hydroxyfunktion modifiziert oder substituiert worden ist, handelt,
wobei das w/w-Verhältnis (a)/(b) der Zusammensetzung 1,1 bis 1,7 beträgt,
zur Konservierung von Mikroorganismen.

13. Verfahren zur Aufbewahrung von Mikroorganismen, umfassend die folgenden Schritt:
Herstellen einer Zusammensetzung, die Folgendes umfasst:
(a) mindestens einen nichtreduzierenden Zucker,
(b) Inosit oder ein Derivat davon, wobei es sich bei dem Derivat um ein Inosit, in dem mindestens eine Hydroxyfunktion modifiziert oder substituiert worden ist, handelt,
wobei das w/w-Verhältnis (a)/(b) der Zusammensetzung 1,1 bis 1,7 beträgt,
- Zugeben der Zusammensetzung zu Mikroorganismen, wodurch man eine Mikroorganismen umfassende Zusammensetzung erhält,
- Durchführen eines Gefrier-, Trocknungs- oder Gefriertrocknungsschritts mit der Mikroorganismen umfassenden Zusammensetzung.

## Revendications

1. Composition comprenant :
(a) au moins un sucre non réducteur,
(b) de l'inositol ou un dérivé de celui-ci, où ledit dérivé est un inositol dans lequel au moins une fonction hydroxyle a été modifiée ou substituée,
où le rapport p/p (a)/(b) de ladite composition va de 1,1 à 1,7, et
(c) des microorganismes.

2. Composition selon la revendication 1, dans laquelle les microorganismes sont des bactéries.

3. Composition selon la revendication 1, dans laquelle les microorganismes sont des bactéries lactiques.

4. Composition selon la revendication 1, 2 ou 3, dans laquelle ledit rapport p/p (a)/(b) va de 1,3 à 1,7.

5. Composition selon l'une quelconque des revendications 1 à 4, dans laquelle ledit (a) est un mélange de sucres non réducteurs.

6. Composition selon l'une quelconque des revendications 1-5, dans laquelle ledit ou lesdits sucres non réducteurs sont choisis dans le groupe constitué par le tréhalose, le 2,3,2',3'-tétra-O-benzyl-6,6'-di-O-décanoyl-4,4'-bis-O-(diphénylphosphono)-alpha,alpha-tréhalose, le 6,6'-di-O-décanoyl-4,4'-di-O-phosphono-alpha,alpha-tréhalose, le 6,6'-ester d'acide gras et de 2,3,2',3'-tétra-O-benzyl-4,4'-bis-O-(diphényl-phosphono)-alpha,alpha-tréhalose, le saccharose, le saccharose-6-benzoate, le saccharose-6-acétate, le saccharose-6-glutarate et le saccharose-6-laurate.

7. Composition selon l'une quelconque des revendications 1 à 4, dans laquelle ledit (a) est le tréhalose et ledit (b) est l'inositol.

8. Composition selon la revendication 3, dans laquelle lesdites bactéries lactiques sont choisies dans le groupe constitué par les souches des genres *Lactococcus, Lactobacillus, Leuconostoc, Bifidobacterium, Carnobacterium, Enterococcus, Propionibacterium, Pediococcus, Streptococcus,* et des mélanges de celles-ci.

9. Composition selon la revendication 8, dans laquelle lesdites bactéries lactiques sont choisies dans le groupe constitué par les souches des espèces et sous-espèces *Bifidobacterium bifidum, Bifidobacterium lactis, Bifidobacterium longum, Bifidobacterium breve, Lactobacillus reuteri, Lactobacillus acidophilus, Lactobacillus casei, Lactobacillus plantarum, Lactobacillus delbrueckii bulgaricus, Lactobacillus rhamnosus, Streptococcus thermophilus, Lactococcus lactis, Lactobacillus pentoceus, Lactobacillus buchneri, Lactobacillus brevis, Pediococcus pentosaceus, Pediococcus acidilactici, Pediococcus parvulus, Propionibacterium freudenreichii, Propionibacterium jensenii, Streptococcus salivarius,* et des mélanges de celles-ci.

10. Composition selon l'une quelconque des revendications 1, 2, 3, 8 et 9, où ladite composition comprend des microorganismes selon une concentration allant de 1×10⁸ UFC/g de composition à 5×10¹² UFC/g de composition.

11. Composition selon l'une quelconque des revendications 1, 2, 3, et 8-10, où ladite composition comprend :
(a) au moins 4% en poids dudit sucre non réducteur, et
(b) au moins 3% en poids dudit inositol ou d'un dérivé de celui-ci.

12. Utilisation d'une composition comprenant :
(a) au moins un sucre non réducteur,
(b) de l'inositol ou un dérivé de celui-ci, où ledit dérivé est un inositol dans lequel au moins une fonction hydroxyle a été modifiée ou substituée,
où le rapport p/p (a)/(b) de ladite composition va de 1,1 à 1,7,
pour la conservation de microorganismes.

13. Méthode de conservation de microorganismes, comprenant les étapes suivantes :
- la préparation d'une composition comprenant
(a) au moins un sucre non réducteur,
(b) de l'inositol ou un dérivé de celui-ci, où ledit dérivé est un inositol dans lequel au moins une fonction hydroxyle a été modifiée ou substituée,
où le rapport p/p (a)/(b) de ladite composition va de 1,1 à 1,7,
- l'addition de ladite composition à des microorganismes, afin d'obtenir une composition comprenant des microorganismes,
- la soumission de ladite composition comprenant des microorganismes à une étape de congélation, séchage ou lyophilisation.
